# EUROPEAN PATENT SPECIFICATION

(11) **EP 3 349 850 B1**
(45) Date of publication and mention of the grant of the patent: **23.10.2019**
(21) Application number: 16770652.2
(22) Date of filing: 16.09.2016
(51) Int. Cl.: A61N 1/378, A61N 1/375

(54) **ASSEMBLY TECHINIQUES FOR SINTERED ANODES AND CATHODES**
MONTAGEVERFAHREN FÜR GESINTERTE ANODEN UND KATHODEN
TECHNIQUES D'ASSEMBLAGE POUR ANODES ET CATHODES FRITTÉES

(30) Priority: 16.09.2015 US 201562219278 P
(43) Date of publication of application: 25.07.2018
(60) Divisional application: 19190996.9
(73) Proprietor: Cardiac Pacemakers, Inc., St. Paul, MN 55112-5798 (US)
(72) Inventor: SHERWOOD, Gregory J., North Oaks, Minnesota 55127 (US); KUHN, Peter Jay, Crystal, MN 55422 (US); OLSON, Jaymes, Lino Lakes, MN 55014 (US); ANDERSON, Matthew, Coon Rapids, Minnesota 55448 (US); WAYTASHEK, Brian V., Lino Lakes, Minnesota 55014 (US); FLOETER, Aaron, Waconia, MN 55387 (US)
(74) Representative: Peterreins Schley
(86) International application number: PCT/US2016/052096
(87) International publication number: WO 2017/049063

(56) References cited:
- US-A1- 2011 152 958
- US-A1- 2011 152 959
- US-A1- 2011 152 960
- US-A1- 2011 317 370
- US-A1- 2013 041 420

## Description

### TECHNICAL FIELD

This document relates generally to energy storage and particularly to sintered electrodes to store energy in an implantable medical device.

### BACKGROUND

Electrical stimulation therapy has been found to benefit some patients. For example, some patients suffer from an irregular heartbeat or arrhythmia and may benefit from application of electrical stimulation to the heart. Some patients suffer from a particular type of arrhythmia called a fibrillation. Fibrillations may affect different regions of the heart, such as the atria or the ventricles. When a fibrillation occurs in the ventricles, the heart's ability to pump blood is dramatically reduced, putting the patient at risk of harm. It has been found that applying an electrical stimulation to the patient can effectively treat patients suffering disorders such as from fibrillation by restoring a regular heartbeat.

Because disorders such as fibrillations can happen at any time, it is helpful to have a device that is easily accessible to treat them. In some cases, it is helpful if that device is portable or implantable. In developing a device that is portable or implantable, it is helpful to have access to components that are compact and lightweight and that can perform to desired specifications.

US 2011/152958 A1 relates generally to energy storage and particularly to sintered electrodes to store energy in an implantable medical device.

US 2013/041420 A1 relates generally to energy storage and particularly to sintered electrodes including a 3-Dimensional ("3D") framework.

US 2011/317370 A1 relates generally to energy storage and particularly to at least one electronic component mounted on an electrode such as a capacitor electrode used in a cardiac defibrillator.

US 2011/152959 A1 relates generally to energy storage and particularly to sintered electrodes to store energy in an implantable medical device.

US 2011/152960 A1 relates generally to energy storage and particularly to sintered electrodes to store energy in an implantable medical device.

### SUMMARY

The invention is defined in the appended claims. Aspects, embodiments and examples disclosed herein which do not fall within the scope of the appended claims do not form part of the invention, and are merely provided for illustrative purposes.
In example 1, an apparatus includes a capacitor case sealed to retain electrolyte; a plurality of sintered anodes disposed in the capacitor case, each of the plurality of sintered anodes having a hole at least partially through the sintered anode; and at least one post through the holes of the plurality of sintered anodes to electrically couple the sintered anodes.
In example 2, the subject matter of claim 1 can optionally include each of the holes in the plurality of anodes being a threaded hole.
In example 3, the subject matter of claims 1 or 2 can optionally include two threaded posts used to interconnect the plurality of sintered anodes.
In example 4, the subject matter of any of claims 1-3 can optionally include one of the plurality of anodes including a second hole configured to receive a threaded feedthrough post.
In example 5, a method of assembling a capacitor includes sintering cathode material directly to an inside surface of a capacitor case, the cathode material forming one or more sintered cathodes having a shape; and placing a sintered anode over or around the sintered cathodes, the sintered anode having one or more mating portions that match the shape of the one or more sintered cathodes such that the mating portions matingly receive the sintered cathodes.
In example 6, the subject matter of example 5 can optionally include the mating portions of the sintered anode being shaped like a fin and the sintered cathode having a fin shape.
In example 7, the subject matter of example 5 can optionally include the sintered cathode having a cylindrical shape and the mating portions of the sintered anode including rounded cut-outs at one or more corners of the sintered anode.
In example 8, the subject matter of example 5 can optionally include, the mating portions being conical and the sintered cathode being conical.
In example 9, the subject matter of any of examples 5-8 can optionally include the cathode including a bed of nails structure.
In example 10, the subject matter of any of examples 5-9 can optionally include a separator applied to the cathode, wherein the separator includes a high dielectric polymer directly applied to an outer surface of the sintered cathode.
In example 11, a method of assembling a capacitor includes sintering cathode material directly to a PCB within a capacitor case, the cathode material forming one or more sintered cathodes having a shape; and placing a sintered anode over the sintered cathodes, the sintered anode having one or more mating portions that match the shape of the one or more sintered cathodes such that the mating portions matingly receive the sintered cathodes.
In example 12, the subject matter of example 11 can optionally include the mating portions of the sintered anode being shaped like a fin and the sintered cathode having a fin shape.
In example 13, the subject matter of claim 12 can optionally include the sintered cathode having a cylindrical shape and the mating portions of the sintered anode including rounded cut-outs at one or more corners of the sintered anode.
In example 14, the subject matter of example 11 can optionally include the mating portions being conical and the sintered cathode being conical.
In example 15, a method of assembling a capacitor includes sintering cathode material onto a aluminum foil, the cathode material forming one or more sintered cathodes having a shape; and placing a sintered anode over the sintered cathodes, the sintered anode having one or more mating portions that match the shape of the one or more sintered cathodes such that the mating portions matingly receive the sintered cathodes; and applying a polymer coating over the sintered anode, the sintered cathode and the aluminum foil.

This Summary is an overview of some of the teachings of the present application and not intended to be an exclusive or exhaustive treatment of the present subject matter. Further details about the present subject matter are found in the detailed description and appended claims. Other aspects of the invention will be apparent to persons skilled in the art upon reading and understanding the following detailed description and viewing the drawings that form a part thereof. The scope of the present invention is defined by the appended claims and their legal equivalents.

### BRIEF DESCRIPTION OF THE DRAWINGS

The drawings illustrate generally, by way of example, various embodiments discussed in the present document. The drawings are for illustrative purposes only and may not be to scale.
FIG. 1 shows a schematic representation of a medical system including a sintered capacitor, in accordance with one embodiment.
FIG. 2 shows an implanted medical system including a sintered capacitor, in accordance with one embodiment.
FIG. 3 shows a capacitor, in accordance with one embodiment.
FIG. 4 shows a detail of the capacitor of FIG. 3, in accordance with one embodiment.
FIG. 5 shows a detail of the capacitor of FIG. 3, in accordance with one embodiment.
FIG. 6 shows a detail of the capacitor of FIG. 3, in accordance with one embodiment.
FIG. 7 shows a detail of the capacitor of FIG. 3, in accordance with one embodiment.
FIG. 8 shows a capacitor, in accordance with one embodiment.
FIG. 9 shows a capacitor, in accordance with one embodiment.
FIG. 10 shows a capacitor, in accordance with one embodiment.
FIG. 11 shows a capacitor, in accordance with one embodiment.
FIG 12 shows an anode, in accordance with one embodiment.
FIG. 13 shows a capacitor, in accordance with one embodiment.
FIG. 14 shows a capacitor, in accordance with one embodiment.

### DETAILED DESCRIPTION

The following detailed description of the present invention refers to subject matter in the accompanying drawings which show, by way of illustration, specific aspects and embodiments in which the present subject matter may be practiced. These embodiments are described in sufficient detail to enable those skilled in the art to practice the present subject matter. References to "an", "one", or "various" embodiments in this disclosure are not necessarily to the same embodiment, and such references contemplate more than one embodiment. The following detailed description is, therefore, not to be taken in a limiting sense, and the scope is defined only by the appended claims, along with the full scope of legal equivalents to which such claims are entitled.

This document concerns sintered electrodes for use in an electrical energy storage device. Specific examples include sintered anodes formed of aluminum or its alloys. Certain examples are for use in aluminum electrolytic capacitors. Additional benefits stem from an increased surface area that is a product of sintering.

Sintering results in many interstices (i.e., spaces) between grains of the electrode. Sintered electrodes resemble crushed grains with interstices between the grains. The interstices are filled with electrolyte, thereby increasing capacitance per unit of volume, as capacitance is proportional to a surface area exposed to electrolyte. An electrode with such interstices offers improved lateral or parallel movement of electrons in relation to a major surface of a flat electrode layer, as etched electrodes restrict lateral movement because the etchings result in voids that are typically perpendicular to the major surface of the flat layer. Accordingly, some examples have a lower ESR (equivalent series resistance) compared to etched foils due to this enhance ionic flow.

Overall, an energy storage device using the sintered electrodes described here is well suited for use in an implantable medical device such as a defibrillator. Because sintering can produce a variety of shapes, sintered electrodes can be used to create energy storage devices such as capacitors that have custom shapes versus simple rolled cylinders or a prism having a parallelogram as its base. Further, manufacturing efficiency is improved, by easing the steps and parts in manufacturing a capacitor and by reducing waste. The interstices are very small, making the electrodes rigid and able to withstand handling by a machine or assembly personnel. These electrodes demonstrate an improved energy density over etched electrodes and are therefore useful to make smaller implantable devices that are able to deliver an amount of energy for a particular therapy.

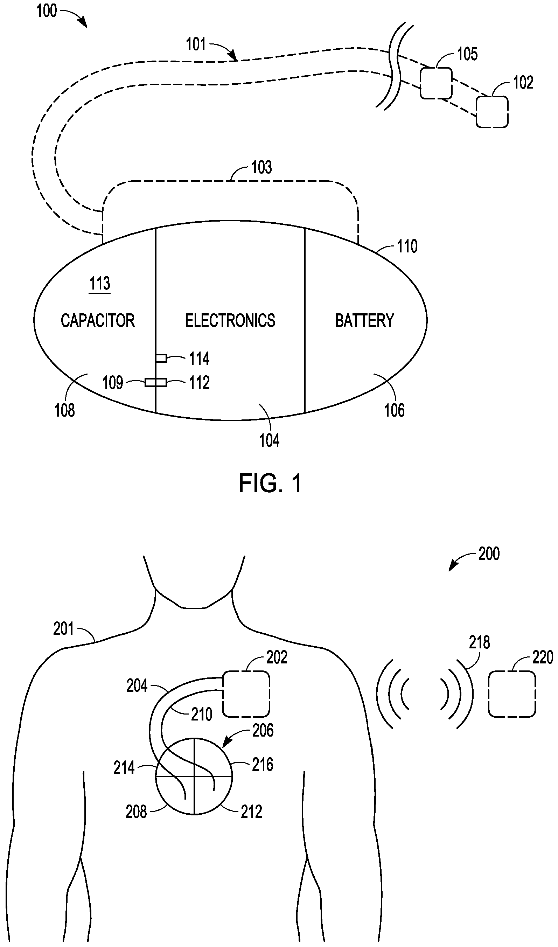

FIG. 1 is a schematic of a medical system 100 including a sintered capacitor, according to some embodiments. The medical system 100 represents any number of systems to provide therapeutic stimulus, such as to a heart. Examples of medical systems include, but are not limited to, implantable pacemakers, implantable defibrillators, implantable nerve stimulation devices and devices that provide stimulation from outside the body, including, but not limited to, external defibrillators.

Electronics 104 are to monitor the patient, such as by monitoring a sensor 105, and to monitor and control activity within the system 100. In some examples, the electronics 104 are to monitor a patient, diagnose a condition to be treated such as an arrhythmia, and control delivery of a stimulation pulse of energy to the patient. The electronics 104 can be powered wirelessly using an inductor. Alternatively, the electronics 104 can be powered by a battery 106. In some examples, electronics 104 are to direct small therapeutic bursts of energy to a patient from the battery 106.

For therapies, such as defibrillation, that use energy discharge rates exceeding what battery 106 is able to provide, a capacitor 108 is used. Energy from the battery 106 is controlled by the electronics 104 to charge the capacitor 108. The capacitor 108 is controlled by the electronics 104 to discharge to a patient to treat the patient. In some examples, the capacitor 108 completely discharges to a patient, and in additional examples, the capacitor is switched on to provide therapeutic energy and switched off to truncate therapy delivery.

Some examples of a medical system 100 include an optional lead system 101. In certain instances, after implantation, the lead system 101 or a portion of the lead system 101 is in electrical communication with tissue to be stimulated. For example, some configurations of lead system 101 contact tissue with a stimulation electrode 102. The lead system 101 couples to other portions of the system 100 via a connection in a header 103. Examples of the system 101 use different numbers of stimulation electrodes and/or sensors in accordance with the needs of the therapy to be performed.

Additional examples function without a lead 101. Leadless examples can be positioned in contact with the tissue to be stimulated, or can be positioned proximal to tissue to shock the tissue to be stimulated through intermediary tissue. Leadless examples can be easier to implant and can be less expensive as they do not require the additional lead components. The housing 110 can be used as an electrode in leadless configurations.

In certain embodiments, the electronics 104 include an electronic cardiac rhythm management circuit coupled to the battery 106 and the capacitor 108 to discharge the capacitor 108 to provide a therapeutic pulse, such as a defibrillation pulse. In some examples, the system 100 includes an anode and a cathode sized to deliver a pulse of at least approximately 50 joules. Other configurations can deliver larger amounts of energy. Some configurations deliver less energy. In some examples, the energy level is predetermined to achieve a delivered energy level mandated by a governing body or standard associated with a geographic region, such as a European country. In an additional embodiment, the anode and cathode are sized to deliver a defibrillation pulse of at least approximately 60 joules. In some examples, this is the energy level is predetermined to achieve an energy level mandated by a governing body of another region, such as the United States. In some examples, electronics 104 are to control discharge of a defibrillation pulse so that the medical system 100 delivers only the energy mandated by the region in which the system 100 is used.

One characteristic of some sintered electrode examples is that at least one anode and a cathode have a DC capacitance that is approximately 23% greater than a AC capacitance for the at least one anode and the cathode of an etched capacitor that has 74.5 microfarads per cubic centimeter. In some examples, the at least one anode and the cathode have an AC capacitance of at least 96.7 microfarads per cubic centimeter at 445 total voltage. In some examples, this is comparable to an operating voltage of about 415 volts. This is a 30% improvement over an etched capacitor that has 74.5 microfarads per cubic centimeter. Total voltage is the voltage that allows 1 milliamp of leakage per square centimeter for an electrode. Some examples are aged to 415 volts.

In certain examples, the capacitor 108 includes a capacitor case 113 sealed to retain electrolyte. In some examples, the capacitor case 113 is welded. In some instances, the capacitor case 113 is hermetically sealed. In additional examples, the capacitor case 113 is sealed to retain electrolyte, but is sealed with a seal to allow flow of other matter, such as gaseous diatomic hydrogen or a helium molecule. Some of these examples use an epoxy seal. The capacitor further includes a conductor 109 coupled to one of the electrodes of the capacitor 108. The conductor 109 sealingly extends through the capacitor case to a first terminal 112 disposed on an exterior of the capacitor case 113. A second terminal 114 can be disposed on the exterior of the capacitor case 113 and in electrical communication with the other electrode of the capacitor 108. The first terminal 112 and the second terminal 114 are electrically isolated from one another.

A hermetically sealed device housing 110 is used to house components, such as the battery 106, the electronics 104, and the capacitor 108. Hermeticity is provided by welding components into the hermetically sealed device housing 110, in some examples. Other examples bond portions of the housing 110 together with an adhesive such as a resin based adhesive such as epoxy. Accordingly, some examples of the housing 110 include an epoxy sealed seam or port. Several materials can be used to form housing 110, including, but not limited to, titanium, stainless steel, nickel, a polymeric material, or combinations of these materials. In various examples, the housing 110 and the case 113 are biocompatible.

The capacitor 108 is improved by the present electrode technology in part because it can be made smaller and with less expense and a variety of shapes and configurations. The improvement provided by these electrodes is pertinent to any application where high-energy, high-voltage, or space-efficient capacitors are desirable, including, but not limited to, capacitors used for photographic flash equipment. The present subject matter extends to energy storage devices that benefit from high surface area sintered electrodes including, but not limited to, aluminum. The electrodes described here can be incorporated into cylindrical capacitors that are wound, in addition to stacked capacitors.

FIG. 2 is an implanted medical system 200, implanted in a patient 201, and including a sintered capacitor, according to some embodiments. The system includes a cardiac rhythm management device 202 coupled to a first lead 204 to extend through the heart 206 to the right ventricle 208 to stimulate at least the right ventricle 208. The system also includes a second lead 210 to extend through the heart 206 to the left ventricle 212. In various embodiments, one or both of the first lead 204 and the second lead 210 include electrodes to sense intrinsic heart signals and to stimulate the heart. The first lead 204 is in direct contact (e.g., touching) with the right atrium 214 and the right ventricle 208 to sense and/or stimulate both those tissue regions. The second lead 210 is in direct contact with the left atrium 216 and the left ventricle 212 to sense and/or stimulate both those tissue regions. The cardiac rhythm management device 202 uses the lead electrodes to deliver energy to the heart, either between electrodes on the leads or between one or more lead electrodes and the cardiac rhythm management device 202. In some examples, the cardiac rhythm management device 202 is programmable and wirelessly communicates 218 programming information with a programmer 220. In some examples, the programmer 220 wirelessly 218 charges an energy storage device of the cardiac rhythm management device 202.

The present system allows for different concepts for the design of high voltage aluminum electrolytic capacitors. As will be discussed, the present system allows for reducing assembly time and cost by providing shapes that allow for ease of assembly with reduction of precise robotic assembly.

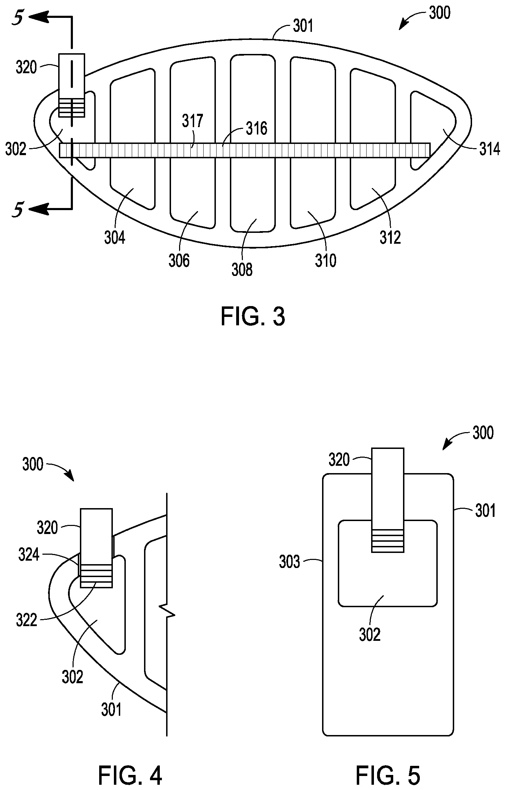

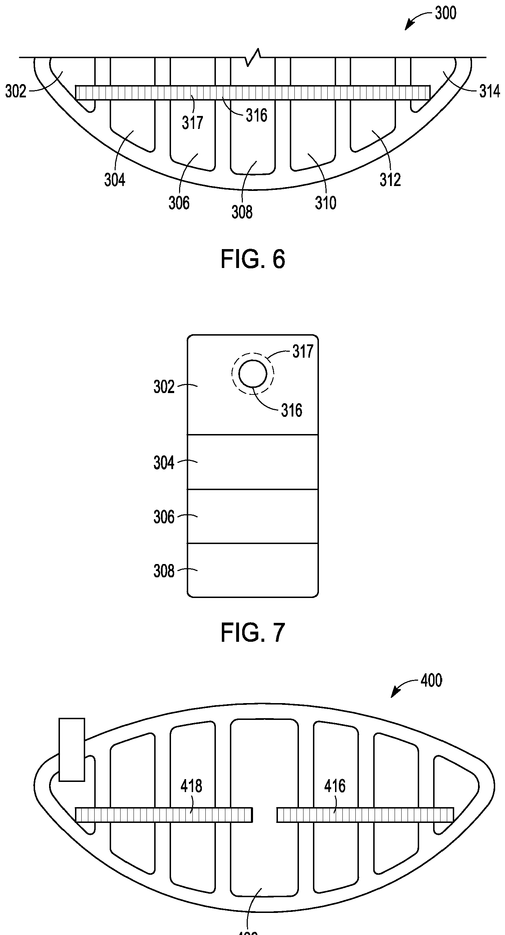

FIGs 3-7 show a capacitor 300, in accordance with one embodiment. FIG. 3 shows a top view of the capacitor 300. FIG. 4 shows a detail of a feedthrough 320 for the capacitor 300. FIG. 5 shows a side view of FIG. 4. FIG. 6 shows a detail of a connection post 316 for the capacitor 300. FIG. 7 is a side view of FIG. 6.

The capacitor 300 generally includes a capacitor case 301 which can be sealed to retain electrolyte and a plurality of sintered anodes 302-314. The anodes 302-314 each have a hole 317 at least partially through the sintered anode 302-314. An interconnect post, such as a threaded interconnect post 316 can be placed through the holes 317 of the plurality of sintered anodes 302-314 to electrically couple the sintered anodes. In one example, each of the holes 317 can be a threaded hole, configured to match the thread of post 316. In an example, each of the anodes 302-314 are made of sintered aluminum and the threaded post 316 is made from aluminum. This system allows the interconnection to be made between the anodes with requiring the welding of tabs or other structures. In one example, the case 301 can be cathodic. Optionally, a sintered cathode can be formed to cover the anodes with a separator there between. For example an e-spun polymer can be coated on at least a portion of the cathode. Other examples, as discussed below, can also be used.

FIGs 4 and 5 show further details of the feedthrough 320 for capacitor 300. In this example, anode 302 includes a second hole 322. A threaded feedthrough post 320 is connected to the anode at hole 322. In one example, hole 322 can be a threaded hole. The feedthrough post 320 extends through case 301 at an opening 324, where the post can be electrically insulated form the case 301. A cover 303 can be welded to case 310.

FIGs 6-7 show details of connection post 316 extending through holes 317 of the anodes 302-314.

FIG. 8 shows another interconnection configuration for the capacitor 300. In this example, two connection post 416 and 418, such as threaded connection posts are utilized. At least one anode 420 does not have a connection hole all the way through the anode, but instead has two partial holes two receive an end of post 418 and an end of post 416. In other embodiments, multiple holes and connection rods can be utilized, depending on need.

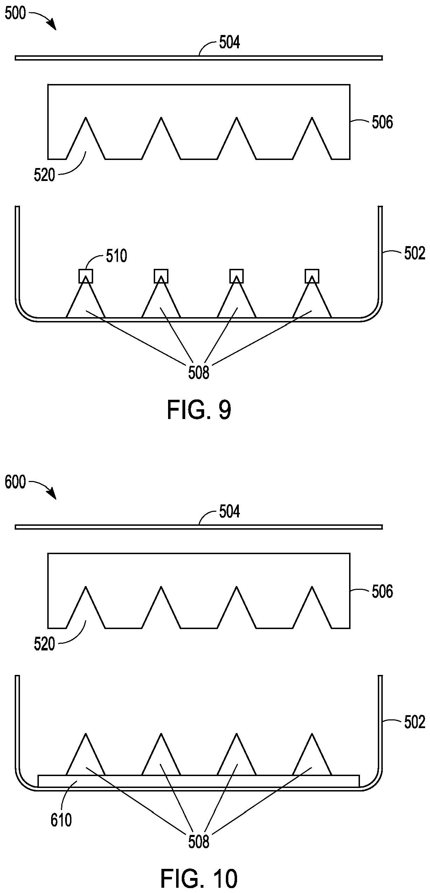

FIG. 9 shows a side view of a capacitor 500 in accordance with one embodiment. Capacitor 500 generally includes a case 502, a lid 504, an anode 506 and cathodes 508. Capacitor 500 allows for ease of manufacturing of capacitors since the case 502 itself is used as an assembly device.

For example, to assemble the capacitor 500, cathode material can be sintered directly to the inside surface of a capacitor case 502, the sintered cathode material forming one or more sintered cathodes 508 having a given shape. Then, the sintered anode 520 can be placed over or around the sintered cathodes 508. The sintered anode 506 can include one or more mating portions such as voids 520 that match the shape of the one or more sintered cathodes 508 such that the voids 520 matingly receive the sintered cathodes 508.

In one example, the mating portions of the sintered anode 506 can shaped like a fin and the sintered cathode 508 can have a fin shape. In one example, the void shape 520 can be conical and the sintered cathode 508 can be conical. In one example, the cathode 508 can include a bed of nails structure and a mating anode can be dropped over the bed of nails. Such shapes and structures allow for ease of assembly with lower complexity. In an example, a separator 510 can be applied to the cathode 508. For example, the separator 510 can include a high dielectric polymer directly applied to an outer surface of the sintered cathode.

As described above for FIG. 1, after assembling the cathodes and the anodes a conductor can be coupled to the sintered anode, with the conductor sealingly extending through the capacitor case to a terminal disposed on an exterior of the capacitor case. A second terminal can disposed on the exterior of the capacitor case and in electrical communication with the sintered cathode, with the terminal and the second terminal electrically isolated from one another.

FIG. 10 shows a capacitor 600, similar to capacitor 500, discussed above. Here the capacitor 600 includes sintering the cathode material directly to a PCB 610 that is within the capacitor case 502. The cathode material forms one or more sintered cathodes 508 having a desired shape. The sintered anode 506 can be placed over the sintered cathodes 508 such that the void spaces 520 match the shape of the one or more sintered cathodes 508 such that the voids matingly receive the sintered cathodes 508. Again, a separator such as discussed above can be placed on the cathode 508

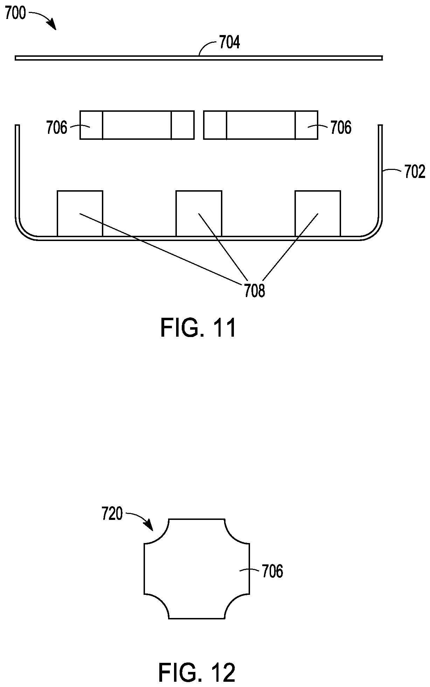

FIG. 11 shows an example of a capacitor 700 configured to allow for ease of manufacturing of capacitors since the case 702 itself is used as an assembly device. Here the capacitor includes the case 702, a lid 704, sintered anodes 706 and sintered cathodes 708. FIG. 12 shows a top view of the anode 706. The anodes 706 include a mating portion, such as a void space 720 comprising a rounded cut-out at one or more corners of the sintered anode 706. The cathodes 708 can have a cylindrical shape and can include a high dielectric polymer directly applied to an outer surface of the sintered cathode 706. To assemble, the cylindrical cathodes 708 are sintered directly to the surface of the case 702 and the anodes 706 are placed within the case like puzzle pieces such that the void spaces 720 matingly receive the sintered cathodes 708.

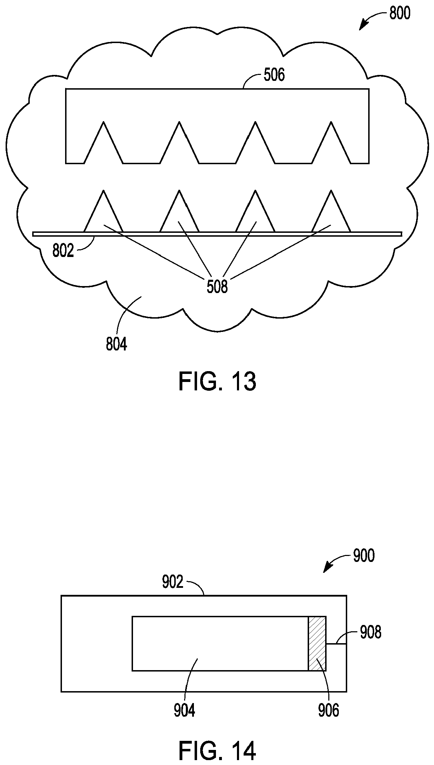

FIG. 13 shows a capacitor 700 including the anode 506 with mating portions including the void spaces 520 and the cathode 508. Here, the capacitor 700 is formed by sintering cathode material onto an aluminum foil 802, the cathode material forming one or more sintered cathodes 508 having a desired shape. The anode 506 is placed over the cathode 508, as discussed above. In this example, a polymer coating 804 is applied over the sintered anode 506, the sintered cathode 508 and the aluminum foil 802.

FIG. 14 shows a capacitor 900 including a case 902 and a sintered anode 904 disposed within the case 902. The sintered anode includes a masked, unsintered portion 906 configured for attaching the sintered anode 904 to a conductor 908, by welding for example. In other examples the anode can be directly attached to the case wall. In some examples, the conductor 908 can be attached to a feedthrough. In other examples, the conductor 908 can be a feedthrough itself. This structure eliminates the need for tabs for the anodes of capacitor 900.

This application is intended to cover adaptations or variations of the present subject matter. It is to be understood that the above description is intended to be illustrative and not restrictive. The scope of the present subject matter should be determined with reference to the appended claims, along with the full scope of legal equivalents to which such claims are entitled.

## Claims

1. An apparatus, comprising:
a capacitor case (301) sealed to retain electrolyte;
a plurality of sintered anodes (302-314) disposed in the capacitor case,
**characterized in that**
each of the plurality of sintered anodes having a hole (317) at least partially through the sintered anode, wherein each of the holes in the plurality of anodes is a threaded hole; and
at least one threaded post (316; 416; 418) placed through the holes of the plurality of sintered anodes to electrically couple the sintered anodes.

2. The apparatus of any of claims 1-2, wherein the plurality of sintered anodes are interconnected by two threaded posts.

3. The apparatus of any of claims 1-2, wherein one of the plurality of anodes includes a second hole (324) configured to receive a threaded feedthrough post (320).

## Patentansprüche

1. Vorrichtung, umfassend:
Kondensatorgehäuse (301), das zum Halten von Elektrolyt versiegelt ist,
eine Vielzahl von in dem Kondensatorgehäuse angeordneten gesinterten Anoden (302 - 314),
**dadurch gekennzeichnet, dass**
jede der Vielzahl von gesinterten Anoden ein mindestens teilweise durch die gesinterte Anode gehendes Loch (317) hat, wobei jedes der Löcher in der Vielzahl von Anoden ein Gewindeloch ist, und
mindestens ein Gewindepfosten (316; 416; 418) durch die Löcher der Vielzahl von gesinterten Anoden platziert ist, um die gesinterten Anoden elektrisch zu koppeln.

2. Vorrichtung nach einem der Ansprüche 1 - 2, wobei die Vielzahl von gesinterten Anoden über zwei Gewindepfosten miteinander verbunden sind.

3. Vorrichtung nach einem der Ansprüche 1 - 2, wobei eine der Vielzahl von Anoden ein zweites Loch (324) aufweist, das dazu ausgestaltet ist, einen Gewindedurchführungspfosten (320) aufzunehmen.

## Revendications

1. Appareil comprenant :
une enveloppe de condensateur (301) scellée de manière à retenir un électrolyte ;
une pluralité d'anodes frittées (302-314) disposées dans l'enveloppe de condensateur,
**caractérisé en ce que**
chacune parmi la pluralité d'anodes frittées présente un trou (317) au moins en partie à travers l'anode frittée, chacun des trous dans la pluralité d'anodes étant un trou fileté ; et
au moins une colonnette filetée (316 ; 416 ; 418) placée à travers les trous de la pluralité d'anodes frittées pour accoupler électriquement les anodes frittées.

2. Appareil selon l'une quelconque des revendications 1 à 2, dans lequel la pluralité d'anodes frittées sont interconnectées par deux colonnettes filetées.

3. Appareil selon l'une quelconque des revendications 1 à 2, dans lequel l'une parmi la pluralité d'anodes comporte un deuxième trou (324) configuré pour recevoir une colonnette d'alimentation filetée (320).
